# EUROPEAN PATENT APPLICATION

(11) **EP 1 270 552 A2**
(43) Date of publication of application: **02.01.2003**
(21) Application number: 02253684.1
(22) Date of filing: 24.05.2002
(51) Int. Cl.: C07C 277/08, C07C 279/12

(54) **Production process for a guanidine derivative containing an amido group and for a salt thereof**

(30) Priority: 25.05.2001 JP 2001157834
(71) Applicant: Ajinomoto Co., Inc., Tokyo 104-8315 (JP)
(72) Inventor: Yumioka, Ryosuke, Ajinomoto Co., Inc. Amino S. L., Kawasaki-shi, Kanagawa 210-0801 (JP); Yokota, Hirofumi, Ajinomoto Co., Inc. Amino S. L., Kawasaki-shi, Kanagawa 210-0801 (JP)
(74) Representative: Nicholls, Kathryn Margaret

(57) **Abstract**

A guanidine derivative containing an amido group, represented by 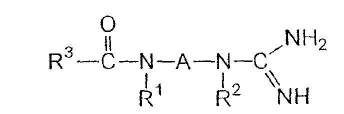 is produced by reacting a guanidine derivative containing an amino group represented by 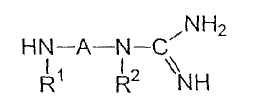 with a fatty acid halide or a fatty acid ester.

## Description

The present invention relates to a production process of a guanidine derivative containing an amido group and its salt. More particularly, this invention relates to a process capable of simply and economically providing a guanidine derivative containing an amido group and its salt without going through a complicated composition process.

Compounds containing a guanidine group are utilized in various fields such as pharmaceuticals, agrochemicals, disinfectant, insecticides, effective metal-collecting agents, chelate agents and the like. Among these, as guanidine derivatives containing an amido group are described in JP-A-H2-243614, JP-A-H4-49221 and JP-A-H4-49222, they have superior properties in comparison with usually used quatery ammonium type cation surfactants, and are utilized as surfactants which impart superior flexibility, moisture retaining property and good finishing feeling to hair.

It is known that a guanidine derivative containing an amido group has superior properties and has been utilized, but its production was not always simple. For example, in JP-A-H6-312972, there are disclosed production processes of a guanidine derivative containing an amido group and its salt. According to the disclosed process, a mono amido amine that is introduced from a diamine is treated with heating under reduced pressure, treated with heating under nitrogen bubbling, or preserved under atmosphere without carbon dioxide, then converted to guanidyl with a cyanamide, S-methylisothiourea and the like, and further, impurities which exist in mixture are removed by procedures such as crystallization and the like.

However, the disclosed process had a reaction step that included a lot of steps and it is complicated. Also, a high level reaction control and a purification process were indispensable in order to minimize dicyanamide, bisamide, urea derivatives and the like which were prepared as by products, and in order to remove them, therefore it was not a simple production process which is not industrially advantageous and which can not be adequately satisfied. Under these circumstances, it has been desired to develop an industrially advantageous process of simply producing a guanidine derivative containing an amido group which is a useful compound.

It is an object of the present invention to provide processes of producing a highly pure guanidine derivative containing an amido group and its salt by an industrially advantageous and simple composition course.

According to one aspect of the invention, a method is provided for the production of a guanidine derivative containing an amido group represented by a general formula (I) 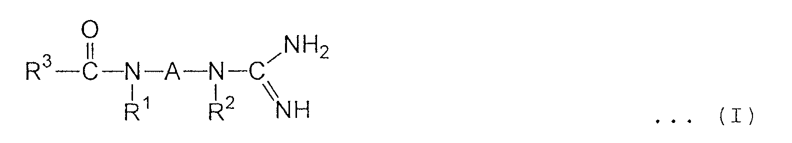 where R¹ and R² are each chosen from a group consisting of hydrogen atoms, a linear chain or branched chain alkyl group having 1 to 4 carbons, and a linear chain or branched chain alkenyl group having up to 4 carbons and R¹ and R² are have same or different composition, R³ is a linear chain or a branched chain alkyl group or an alkenyl group having up to 22 carbons, A is a linear chain or branched chain alkylene group or alkenylene group having up to 10 carbons, the method comprising a step of:
allowing a guanidine derivative containing an amino group represented by the general formula (II) to react with a fatty acid halide or a fatty acid ester, 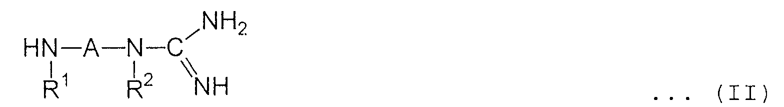
where R¹ and R² are each selected from the group consisting of hydrogen atoms, a linear chain or branched chain alkyl group having 1 to 4 carbons, and a linear chain or branched chain alkenyl group having up to 4 carbons and R¹ and R² are have same or different composition, A is a linear chain or a branched chain alkylene group or an alkenylene group having up to 10 carbons.

Moreover, the salt of a guanidine derivative containing an amido group, according to the present invention is produced by neutralizing the solution of the guanidine derivative containing an amido group.

Other objects and features of this invention will become apparent from the following description of preferred embodiments described, by way of example, with reference to the accompanying drawings, of which:
Fig. 1 is a flow chart which shows the production steps of a recombinant in which Arginine decarboxylase gene is amplified expressed, and
Fig. 2 is a flow chart which shows the construction process of plasmid pTrcadil.

The reaction in the production process of a guanidine derivative containing an amido group of the present invention is represented by the reaction formula below. 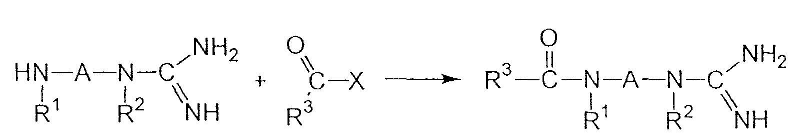 where R¹ and R² are each chosen from a group consisting of hydrogen atoms, a linear chain or branched chain alkyl group having 1 to 4 carbons, and a linear chain or branched chain alkenyl group having up to 4 carbons, and may be the same or different. R³ is a linear chain or branched chain alkyl group or alkenyl group having up to 22 carbons. A is a linear chain or branched chain alkylene group or alkenylene group having up to 10 carbons. X represents halogen or an ester group.

The production process of a guanidine derivative containing an amido group of the present invention will be explained in detail below referring to the attached drawings in an order of
[I] a starting material,
   [A] a fatty acid halide and a fatty acid ester,
   [B] a guanidine derivative containing an amino group, and
[II] reaction conditions.
[I] Starting material

### [A] Fatty acid halide and fatty acid ester

The guanidine derivative containing an amido group of the present invention is obtained by reacting the guanidine derivative containing an amino group with a fatty acid halide or a fatty acid ester. In the present invention, either of the fatty acid halide and the fatty acid ester may be reacted with the guanidine derivative containing an amino group, but the fatty acid halide is preferably used in order to proceed the reaction in a milder reaction condition.

The fatty acid halide used in the present invention has an acyl portion which contains a linear chain or branched chain alkyl group or alkenyl group having 1 to 22 carbons.

Specific examples of the fatty acid halide include acetyl halide, propionyl halide, butyroyl halide, isobutyroyl halide, caproyl halide, octanoyl halide, caproyl halide, lauroyl halide, myristoyl halide, palmitoyl halide, stearoyl halide, isostearoyl halide, oleoyl halide, linoloyl halide, linolenoyl halide, arachidonoyl halide, behenoyl halide, coconut oil fatty acid halide, palm oil fatty acid halide, beef tallow fatty acid halide and the like. In the present invention, the fatty acid halide having an acyl portion which contains a linear chain or branched chain alkyl group or alkenyl group having 7 to 17 carbons is preferable, the fatty acid halide having an acyl portion which contains a linear chain or branched chain alkyl group or alkenyl group having 10 to 15 carbons is more preferable, and the fatty acid halide having an acyl portion which contains a linear chain alkyl group having 11 carbons is most preferable. In particular, a fatty acid chloride is preferable. These can be used alone or 2 or more can be used in combination.

Further, the fatty acid ester used in the present invention is the one having an acyl portion which contains a linear chain or branched chain alkyl group or alkenyl group having 1 to 22 carbons. Specific examples of the fatty acid ester include methyl, ethyl or isopropyl ester and the like or triglyceryl ester of acetic acid, propionic acid, butyric acid, isobutyric acid, caproic acid, octanoic acid, capric acid, lauric acid, myristic acid, palmitic acid, stearic acid, isostearic acid, oleic acid, linolic acid, linoleic acid, arachidonic acid, behenic acid, coconut oil, palm oil, beef tallow and the like. In the present invention, the fatty acid ester having an acyl portion which contains a linear chain or branched chain alkyl group or alkenyl group having 7 to 17 carbons is preferable, the fatty acid ester having an acyl portion which contains a linear chain or branched chain alkyl group or alkenyl group having 11 to 15 carbons is more preferable, and the fatty acid ester having an acyl portion which contains a linear chain alkyl group having 11 carbons is most preferable . In particular, methyl ester is preferable. These can be used alone or 2 or more can be used in combination.

### [B] Guanidine derivative containing amino group

The guanidine derivative containing an amino group used in the present invention can be synthesized by known methods such as an enzyme reaction as described in Can. J. Chem. 1982. 60, 2810-2820, a chemical reaction as described in Z. PHYSIOL. CHEM. 1901. 68, 170-172, and the like, and can be represented by the general formula (II). 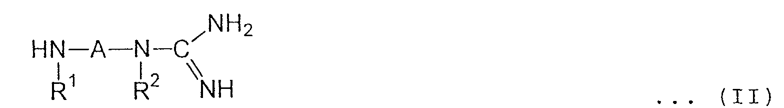 where R¹ and R² are each selected from the group consisting of hydrogen atoms, a linear chain or branched chain alkyl group having 1 to 4 carbons, and a linear chain or branched chain alkenyl group having 1 to 4 carbons, and may be the same or different. R¹ and R² are preferably hydrogen atoms.

A is a linear chain or branched chain alkylene group or alkenylene group having 1 to 10 carbons. A is preferably a linear chain alkenylene group, more preferably a linear chain alkenylene group having 2 to 6 carbons, and preferably a linear chain alkenylene group having 4 carbons, in particular.

Specific examples of the guanidine containing an amino group include 2-aminoethylguanidine, 3-aminopropylguanidine, 4-aminobutylguanidine, 5-aminopentylguanidine, 6-aminohexylguanidine, 8-aminooctylguanidine, 10-aminodecylguanidine and the like. These can be used alone or 2 or more can be used in combination. Among these, 4-aminobutylguanidine (agmatine) can be comparatively simply produced by decarboxylating arginine which is one of amino acids, using Arginine decarboxylase which microorganism produces, and it is most preferable.

The process of producing 4-aminobutylguanidine from arginine using Arginine decarboxylase will be explained below.

It is known that Arginine decarboxylase which is produced by microorganism produces 4-aminobutylguanidine using arginine being one of amino acids as one of substrate as shown in the under-mentioned reaction formula.

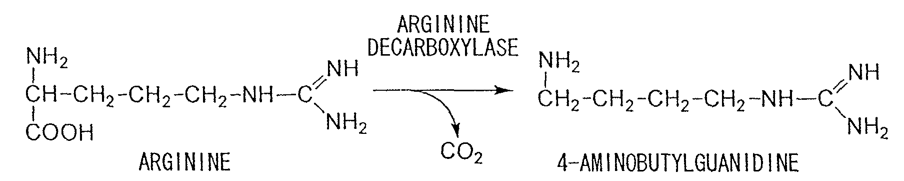

As the microorganism which produces Arginine decarboxylase, Escherichia genus including E. Coli, Salmonella genus (J. Bacteriol., 177, 4097-4104 (1995)), and plants (Mol. Gen. Genet., 224,431-436 (1990), Plant Physiol., 100, 146-152 (1992), Plant Physiol., 103, 829-834 (1993), Plant Cell Physiol., 35, 1245-1249 (1994)) are known. In the present invention, Arginine decarboxylase can obtain these known Arginine decarboxylases from the productive fungus without specific limitation.

Further, in the present invention, a recombinant in which Arginine decarboxylase gene is amplified expressed, or Arginine decarboxylase which is obtained from the recombinant, or Arginine decarboxylase-containing article is preferably used. The term "amplified expressed" referred to herein means that an expression of the gene has been amplified, that is, an amount of expressed protein coded by the gene has been increased.

It may be improve by the adjusting region of Arginine decarboxylase gene in order to raise the expression quantity of Arginine decarboxylase gene. The improvement of the adjusting region means that a transcription quantity of Arginine decarboxylase gene being at a downstream is increased by, for example, newly inserting a strong promoter, by reinforcing a promoter by introduction of variation in the promoter, by controlling the expression quantity of repressor protein which is bonded with the adjusting region, or the like.

In order to raise the expression quantity of Arginine decarboxylase gene, it is preferable to connect Arginine decarboxylase gene with multi copy type vector to prepare a recombinant DNA, and retain the recombinant DNA on a microorganism. When the microorganism which raised the expression quantity of Arginine decarboxylase gene is induced, for example, a requisite gene region is obtained by amplification based on the known gene information of Arginine decarboxylase productive microorganism such as E.Coli using PCR (polymerase chain reaction) method, and is mounted on a vector such as plasmid or the like, and host cell is transformed.

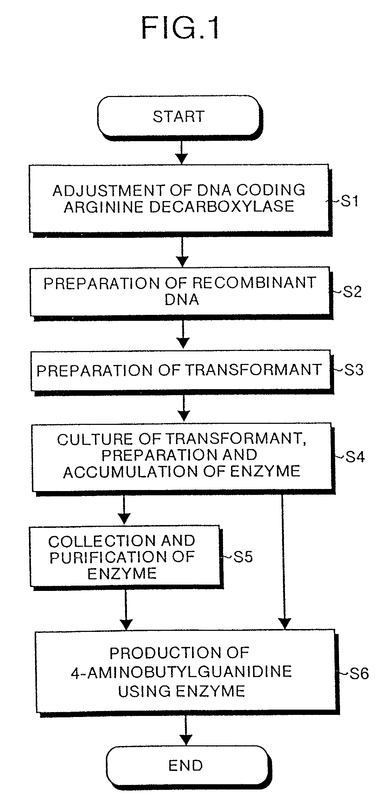

Fig. 1 is a flow chart which shows a production step of a recombinant in which Arginine decarboxylase gene is amplification expressed.

Firstly, DNA coding the Arginine decarboxylase of the present invention is prepared (step S1).

Then, a recombinant DNA is prepared by connecting the Arginine decarboxylase which was prepared, with vector DNA (step S2), and host cell is transformed by the recombinant DNA to prepare a transformant (step S3). Successively, the transformant is cultured in a medium, Arginine decarboxylase is prepared and accumulated in the medium and/or in cell (step S4).

Then, proceeding to step S5, the mass production of Arginine decarboxylase gene is carried out by recovering and purifying the Arginine decarboxylase prepared.

Further, 4-aminobutylguanidine is produced in large quantity by decarboxylating arginine using the medium in which the Arginine decarboxylase produced in the step S5 or Arginine decarboxylase of the step S4 was accumulated (step S6).

A method of producing 4-aminobutylguanidine using the recombinant in which Arginine decarboxylase gene is amplified expressed according to recombinant DNA technology, or Arginine decarboxylase which was obtained from the recombinant, orArginine decarboxylase-containing article, will be explained in detail in an order described below.
(1) Preparation of Arginine decarboxylase gene
(2) Preparation of recombinant DNA
(3) Preparation of recombinant
(4) Preparation and accumulation of Arginine decarboxylase
(5) Production of 4-aminobutylguanidine

### (1) Preparation of Arginine decarboxylase gene

Arginine decarboxylase gene can be obtained without specific limitation from known microorganisms which produce Arginine decarboxylase such as Escherichia genus including E. Coli, Salmonella genus (J. Bacteriol., 177, 4097-4104 (1995)), and plants (Mol. Gen. Genet., 224, 431-436 (1990), Plant Physiol., 100, 146-152 (1992), Plant Physiol., 103, 829-834 (1993), Plant Cell Physiol., 35, 1245-1249 (1994)) and the like.

In particular, 2 kinds of inductive type enzyme (Gene name is adi) and non-inductive type enzyme (Gene name is spe A) are known in E. Coli. When the existing environment of a microorganism is made to be in an extremely acidic condition, namely when acid stress is generated, adi is urgently expressed and induced, decarboxylates arginine to produce basic 4-aminobutylguanidine, neutralization is carried out, and it is understood that it is a mechanism of temporarily escaping the crisis of existence (J. Bacteriol., 177, 4097-4104 (1995), Appl. Environ. Microbiol., 62, 3094-3100 (1996), J. Bacteriol., 181, 3525-3535 (1999)).

It is known concerning speA that there exist speA of forming operon as speAB and coding an enzyme which prepares 4-aminobutylguanidine and carbon dioxide from arginine and speB of coding an enzyme which prepares putrescine and urea and 4-aminobutylguanidine (J. Bacteriol., 174, 758-764 (1992)). It is understood that the present enzyme system is a non-inductive type, and a metabolism process which is required for polyamine biosynthesis of putrescine, spermidine and the like to microorganism, together with Ornithine decarboxylase in which putrescine is prepared by decarboxylating ornithine (Proc. Natl. Acad. Sci. U.S.A., 80, 5181-5184, J. Bacteriol., 173, 3615-3621 (1991), Int. J. Biochem., 26, 991-1001 (1994), J. Bacteriol., 180, 4278-4286 (1998), Microbiology, 145, 301-307 (1999)). The various enzyme chemical properties and gene configuration of the Arginine decarboxylase which codes speaA and agmatinase which codes speB are studied in detail (J. Biol. Chem., 248, 1687-1695 (1973), Methods Enzymol., 94, 117-121 (1983), Gene, 30, 129-136 (1984), J. Bacteriol., 172, 4631-4640 (1990)). In particular, concerning the present enzyme system and Ornithine decarboxylase, the deletion of Ornithine decarboxylase gene and dissociant which deleted agmatinase gene (speB) are prepared (J. Bacteriol., 101, 725-730 (1970), J. Biol. Chem., 254, 12419-12426 (1979), Biochem. J., 234, 617-622 (1986), Proc. Natl. Acad. Sci. U.S.A., 84, 4423-4427 (1987)). The deletion of both enzymes may influence the growth of microorganism, but are not lethal, and physiology in the microorganism of a polyamine is not cleared yet (J. Bacteriol., 101, 731-737 (1970), J. Bacteriol., 113, 271-277 (1973), Adv. Polyamine Res., 4, 495-506 (1983), J. Bacteriol., 163, 522-527 (1985)). However, it known that amines such as 4-aminobutylguanidine and the like are volatile at alkali side, irritable for skin and mucous, and absorbed in body through skin and mucous. Toxicity for microorganism is not cleared, but it can be considered that an amine is alkaline, and becomes lethal to a microorganism if it becomes highly concentrated without neutralization.

In the present invention, adi gene derived from E. Coli is preferably used as Arginine decarboxylase gene, in particular. SpeA is also a gene coding Arginine decarboxylase, and there is an advantageous property that Arginine decarboxylase (J. Biol. Chem., 243, 1671-1677 (1968)) which is coded in adi has a high specific activity and the like in comparison with Arginine decarboxylase (J. Biol. Chem., 248, 1687-1695 (1973)) which is coded in speA.

In order to obtain Arginine decarboxylase gene, for example, adi which is a gene coding Arginine decarboxylase may be cloned from the chromosome DNA of W3110 species (ATCC27325) of E. Coli K1 using the PCR method. Specifically, a DNA molecule having about 30 base pairs is synthesized from the Base Sequence of known adi gene, and this is utilized as a probe and isolated from Gene Library of E. Coli chromosome . The chromosome DNA used at this time may be any strain so far as it is derived from E. Coli.

A method of synthesizing the DNA molecule is disclosed in Tetrahedron Letters, 22, 1859 (1981). Further, the DNA molecule can be synthesized using a synthesizer manufactured by Applied Biosystems Co., Ltd. When the full length of adi gene derived from E. Coli is isolated from Gene Library of E. Coli chromosome, the DNA molecule can be utilized as a probe, and can be used as a primer when the adi gene derived from E. Coli is amplified by the PCR method.

The operation of the PCR method is described in White, T. J., Trends Genet., 5, 185 (1989) and the like. A method of preparing chromosome DNA, and further, a method of isolating the objective DNA molecule from Gene Library are described in Molecular Cloning, 2^{nd} edition, Cold Spring Harbor press (1989) and the like.

Variation type by genetic polymorphism is included in the adi gene used in the present invention. Further, the genetic polymorphism means a phenomenon in which the Amino acid Sequence of a protein is partially varied by natural genetic mutation.

Further, the activity of an enzyme itself may be enhanced by introducing variation in the structural gene itself of Arginine decarboxylase in order to enhance the activity of Arginine decarboxylase.

In order to generate variation in a gene, there are a site specific variation method (Kramer, W., and Frits, H. J., Methods Enzymol., 154, 350 (1987)), a recombinant PCRmethod (PCR Technology, Stockton Press (1989)), a method of chemically synthesizing the DNA of a specific portion, or a method of carrying out the hydroxylamine treatment of the gene, and a method of carrying out the ultraviolet irradiation treatment of strain having the gene or the treatment by chemicals such as nitrosoguanidine, nitrous acid and the like.

### (2) Preparation of recombinant DNA

The recombinant DNA in the present invention means those which were connected with the vector of plasmid and phage DNA using the above-mentioned Arginine decarboxylase gene (adi and the like) as passenger.

The vector is preferably a so-called multi copy type, and plasmid having a replication starting point derived from Col E1, for example, pUC-based plasmid, pBR322-based plasmid, or its derivative is mentioned. The term "derivative" means those which have been subjected to alteration to plasmid by the substitution, deletion, insertion, addition or reverse position of a base, and the like. Further, the alteration called hereby includes variation treatment by a variation agent, UV irradiation and the like, or alteration by natural variation and the like. In addition to these, transposon (Berg, D. E. and Berg, C. M., Bio/Technol., 1, 417 (1983)), and Mu Phage (JP-A-H2-109985) can be also used. The copy number can be also raised by recombining a gene with a chromosome by a method of using plasmid for homology recombination and the like.

At the time, a lac promoter, a trp promoter, a tac promoter, a trc promoter, a PL promoter, and a promoter which functions in other microorganism are preferably used in order to effectively express the useful gene.

Further, it is preferable to link a terminator which is the sequence of transcription termination, at the downstream of a protein gene in order to increase production amount. As the terminator, a T7 terminator, a fd phage terminator, a T4 terminator, a terminator of tetracycline resistant gene, a terminator of E. Coli trpA gene, and the like are mentioned. The transcription amount of a gene may be increased by newly introducing an enhancer.

Further, the vector has preferably a marker such as an ampicillin resistant gene or the like in order to discriminate a transformant. As the plasmid, for example, expression vector having strong promoters such as pUC-based (manufactured by TAKARA SYUZOU Co., Ltd.), pPROK-based (manufactured by Clone Tec Co., Ltd.), pKK233-2 (manufactured by Clone Tec Co., Ltd.) and the like are commercially available.

### (3) Preparation of recombinant

When a recombinant which raised the expression amount of Arginine decarboxylase gene is induced, transformation is carried out by introducing to host cell a recombinant DNA in which Arginine decarboxylase gene (adi and the like) was connected with plasmid and the vector of phage DNA.

As the host cell which is transformed, a microorganism which is expressed by a gene coding Arginine decarboxylase, for example, bacteria cell, radial fungus cell, yeast cell, mold cell, plant cell, animal cell and the like can be used. E. Coli is preferably used, Escherichia coli is more preferably used, and a competent cell such as E. Coli JM109 or the like is preferably used in particular.

The host cell is transformed using the above-mentioned recombinant DNA. As a method of carrying out transformation and a method of selecting a transformant, methods described in Molecular Cloning, 2^{nd} edition, Cold Spring Harbor press (1989) and the like can be adopted.

### (4) Preparation and accumulation of Arginine decarboxylase.

The objective enzyme is prepared and accumulated by culturing a recombinant which was transformed by a recombinant DNA containing Arginine decarboxylase gene (adi and the like) which is obtained by the above-mentioned method. A method of culturing a recombinant which obtained the high expression capability of Arginine decarboxylase gene will be explained below.

As a medium used, an LB medium (Bactro-tryptone 1%, Yeast extract 0.5%, NaCl 1%, Glucose 1%, pH7.0) is often used, but it may be a usual medium which contains a carbon source, a nitrogen source, an inorganic source, and if necessary, other organic source.

As the carbon source, saccharoides such as glucose, lactose, galactose, fructose, arabinose, maltose, xylose, trehalose, ribose, the hydrolysis product of starch or the like, alcohols such as glycerol, mannitol, sorbitol and the like, organic acids such as gluconic acid, fumaric acid, citric acid, succinic acid and the like can be used.

As the nitrogen source, inorganic ammonium salts such as ammonium sulfate, ammonium chloride, ammonium phosphate and the like, organic nitrogen such as the hydrolysis product of soy bean and the like, ammonia gas, aqueous ammonia and the like can be used. As organic trace nutritive element, it is desirable that requisite substances such as various amino acids, vitamins such as vitamin B6 and the like, nucleic acids such as RNA and the like, or yeast extract and the like are appropriately contained.

Additionally, a small amount of calcium phosphate, magnesium sulfate, iron ion, manganese ion and the like are added.

The culture is preferably carried out under aerobic condition for about 12 to 72 hours, with the culture temperature of 20°C to 45°C, and the culture pH preferably controlled at 5 to 8. Further, an inorganic or organic and acidic or alkaline substance and further, ammonia gas and the like can be used for adjusting the pH.

When the expression amount of Arginine decarboxylase exceeds 10% of protein in microorganism in the recombinant, Arginine decarboxylase forms inclusion body, and the activity of Arginine decarboxylase is occasionally lowered, but the formation of the inclusion body can be suppressed by culturing the recombinant at 30°C or less.

The collection by separation of microorganism from a culture solution and the preparation of enzyme solution can be usually carried out by combining a centrifugal separation, an ultrasonic crush, an ion exchange resin method, a sedimentation method and other known methods.

### (5) Production of 4-aminobutylguanidine

In the production of 4-aminobutylguanidine of the present invention, L-arginine is enzymatically decarboxylated using Arginine decarboxylase which was prepared and accumulated by a recombinant in which Arginine decarboxylase gene is amplified expressed.

The method of acting Arginine decarboxylase on L-arginine is not specifically limited, and for example, L-arginine may be directly added in a culture solution while culturing a recombinant in which Arginine decarboxylase gene is amplified expressed, or a microorganism which was separated from a culture solution, a rinsed microorganism and the like may be used. Further, a microorganism-treated article which was obtained by crushing or dissolving the microorganism may be used as it is, and Arginine decarboxylase may be recovered from the microorganism-treated article to be used as a crude enzyme solution. Further, Arginine decarboxylase may be purified to be used. Namely, with a fraction having Arginine decarboxylase activity, all of enzyme and the enzyme-containing article can be used. Wherein "enzyme-containing article" may be well so far as it contains the enzyme, and specifically includes a cultured product, a culture microorganism, a rinsed microorganism, a microorganism-treated article which was obtained by crushing or dissolving a microorganism, a crude enzyme solution, a purified enzyme and the like. A method of directly adding a substrate in a culture solution to be reacted is most preferable from the viewpoint of designing the cost down of the production of 4-aminobutylguanidine by simplifying the steps.

When L-arginine is directly added in a culture solution while culturing a recombinant in which Arginine decarboxylase gene is amplified expressed to proceed the preparation reaction of 4-aminobutylguanidine, the reaction is left alone or carried out while stirring. The reaction temperature is 10°C to 60°C and preferably 25°C to 45°C, and pH is 3 to 8 and pH is preferably controlled at 4.5 to 6. Further, pyridoxal phosphoric acid being a coenzyme is preferably added in the reaction. L-arginine of a substrate may be added so far as the reaction proceeds, and if necessary, the reaction can be carried out at a requisite amount and time.

When the preparation reaction of 4-aminobutylguanidine is carried out using a crude enzyme solution, a culture microorganism is collected by a centrifugal separation operation, then the microorganism is crushed or dissolved, and a crude enzyme solution containing Arginine decarboxylase is prepared. Methods of an ultrasonic crush, a French-press crush, a glass-beads crush and the like can be used for crushing the microorganism. Further, when it is dissolved, egg lysozyme, peptitase treatment or a method of appropriately combining these is used. When the preparation reaction of 4-aminobutylguanidine is carried out using a purified enzyme solution, the crude enzyme solution of Arginine decarboxylase is further purified by usual methods such as sedimentation, filtration, column chromatography and the like. When it is done so, a purification method which utilized the antibody of Arginine decarboxylase can be also utilized.

When the preparation reaction of 4-aminobutylguanidine is proceeded using a crude enzyme solution or a purified enzyme of Arginine decarboxylase, the reaction is proceeded while controlling the reaction solution containing L-arginine of a substrate and the crude enzyme solution or the purified enzyme at 10°C to 60°C and preferably 25°C to 45°C and pH of 3 to 8 and preferably of 4.5 to 6. Pyridoxal phosphoric acid being a coenzyme is preferably added in the reaction. L-arginine of a substrate may be added so far as the reaction proceeds, and if necessary, the reaction can be carried out at a requisite amount and time.

Centrifugal separation, a sedimentation method, a filtration method, an ion exchange resin method, a membrane separation method, and other known methods can be carried out in combination in order to separate and collect a solid insoluble component such as a solid or the like from an enzyme reaction solution which contains 4-aminobutylguanidine. The purified solution containing 4-aminobutylguanidine is set through a step of excluding impurities by an absorbent such as an active carbon treatment or the like, or is utilized as it is. When the containing concentration of 4-aminobutylguanidine is too low, a concentrated solution is made by concentration, or after pH adjustment is carried out with a mineral acid and the like, concentration and crystallization are carried out, and salts of the mineral acids are obtained in accordance with the kind of the mineral acids used.

### [II] Reaction condition

The production process of a guanidine derivative containing an amido group of the present invention can be carried out by adding a fatty acid halide and/or a fatty acid ester to a system in which a guanidine derivative containing an amino group was dissolved or dispersed in a reaction solvent, under a basic condition. 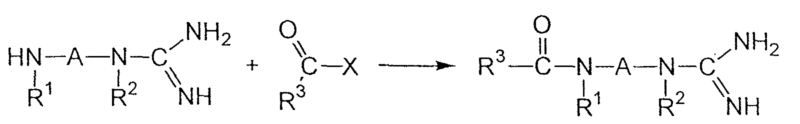 (R¹ and R² are hydrogen atoms, a linear chain or branched chain alkyl group or alkenyl group having 1 to 4 carbons, and may be the same or different. R³ is a linear chain or branched chain alkyl group or alkenyl group having 1 to 22 carbons. A is a linear chain or branched chain alkylene group or alkenylene group having 1 to 10 carbons. X represents halogen or an ester group.)

When the fatty acid halide is used, water or a mix solvent of organic solvents such as acetone, ethanol, methanol, t-butanol, isopropanol and the like, with water is preferably used as the reaction solvent. Further, when the fatty acid ester is used, a mix solvent of organic solvents such as acetone, ethanol, methanol, t-butanol, isopropanol and the like, with water may be used, and the reaction may be carried out without a solvent.

Inorganic bases such as sodium hydroxide, potassium hydroxide, calcium hydroxide, barium hydroxide and the like which are alkaline substances, organic bases such as triethanol amine, triethyl amine, pyridine and the like are added to a system in which a guanidine derivative containing an amino group was dissolved or dispersed in a reaction solvent, and the fatty acid halide is added at a basic condition, preferably at a range of pH of 9.5 to 12.5 to proceed the reaction. At this time, the fatty acid halide can be collectively added, but the reaction is preferably carried out by dropwise addition in order to control the pH of the system. The method of dropwise addition and the time of dropwise addition are not specifically limited, but it is preferably carried out in a range of 0.1 to 3 hours from the viewpoint of production efficiency. When the fatty acid halide is reacted, the temperature of the system is preferably controlled at 0°C to 80°C and preferably 0°C to 50°C in order to prevent the decomposition of the fatty acid halide.

Further, when the fatty acid ester is reacted, since the reaction hardly proceeds in comparison with the fatty acid halide, the fatty acid ester is added to a guanidine derivative containing an amino group, and the reaction is preferably carried out with no solvent while controlling the temperature of the system at 60°C to 200°C and preferably at 80°C to 150°C.

When the prepared guanidine derivative containing an amido group is precipitated out of the system after reaction, it is filtered and washed to be collected. In this instance, it can be easily supposed that the unreacted guanidine derivative containing an amino group remains, and a salt of fatty acid which is generated by decomposition of the fatty acid halide is prepared in the system as a byproduct. But it can be comparatively easily removed by rinsing with water and the like. As a matter of course, water or an organic solvent being the solvent is distilled off after completion of the reaction, and the remained product can be purified according to a conventional method such as crystallization or the like.

As shown in the under-mentioned formula, the guanidine derivative containing an amido group which was obtained by the present production process may be used as salt, if necessary.

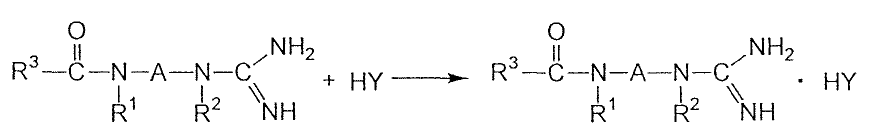

When the guanidine derivative containing an amido group is used as salt, the solution of the guanidine derivative containing an amido group is neutralized with an inorganic acid such as hydrochloric acid, sulfuric acid, phosphoric acid, or the like, or an organic acid such as acetic acid, glutamic acid, asparagic acid, gluconic acid, pyrrolidone carboxylic acid, ascorbic acid, lactic acid, fatty acid, acylglutamic acid or the like, and then can be utilized. Further, when it is used for cosmetics, pharmaceuticals, and other uses under no neutralized condition and the preparations of aqueous solution, emulsion and the like are made, it is appropriately neutralized and can be used.

Embodiments, of the present invention will further be explained, by way of illustration only, in detail below using Examples. However, the present invention is not limited to these Examples.

### Example 1:

### Production of lauroylamidobutylguanidine

### (1) Production of 4-aminobutylguanidine

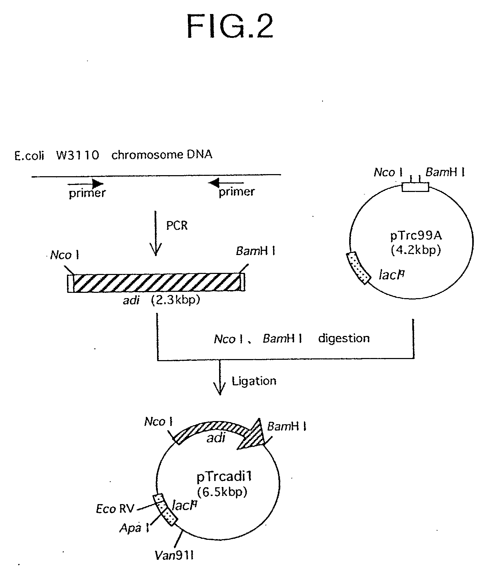

The PCR method (94°C, 30secs., 55°C, 1min., 72°C, 2mins., 30cycles, Gene Amp PCR System Mode 19600 (manufactured by Perkin Elmer Co., Ltd.)) by primers of both terminals which are 30mers and 28mers of GACCATGGCTAAAGTATTAATTGTTGAAAG (Sequence Number 1) and CCGGATCCACGCCTTCAGCGGAATAGTG (Sequence Number 2) which were prepared based on an information which was investigated in E . Coli Gene Bank using "adi" as a keyword, with Pyrobest DNA Polymerase (manufactured by TAKARA SYUZOU Co., Ltd.) was carried out, about 2.3kb fragment of adi structural gene region which covers ATG and SD-ATG and translational termination codon was amplified, and NcoI and BamHI digestion of the fragment was carried out and then, inserted in the NcoI site and BamHI site of pTrc99A (manufactured by Pharmacia K.K.). The present expressed plasmid is named as pTrcadil. (Fig. 2)

The NcoI site to Sequence Number 1 and the BamHI site to Sequence Number 2 are respectively designed in the primer for PCR. Further, the adi which was cloned is expressed in pTrc99A vector under the control of trc promoter, and is translated to Arginine decarboxylase.

Further, a recombinant which transformed E. Coli JM109 by the plasmid pTrcadi1 was cultured in the LB liquid medium (Bacto-tryptone 1%, Yeast extract 0.5%, NaCl 1%, Glucose 0.1%, pH = 7.0) which contains 50mg/L ampicillin and 10mg/L pyridoxine, the expression induction was carried out by adding 10mM arginine (manufactured by AJINOMOTO Co., Inc.) and 1mM IPTG (isopropyl-1-thio-β-D-galactoside), the culture was carried out for about 16 hours and then, a microorganism equivalent to 4ml of broth was collected. These cell bodies were dispersed in 0.4ml of a 0.2M sodium acetate buffer which contains 1% L-Arginine·HCl and 0.02% pyridoxal phosphoric acid, and incubated at 37°C for 1 hour. After the microorganism of the reaction solution was removed by centrifugal operation, the supernatant was analyzed by HPLC, and as a result, 4-aminobutylguanidine was prepared at a conversion rate of almost 100% by the pTrcadi1/JM109 method. pTrcadi1/JM109 i.e. E. coli AJ13839 strain has been deposited as FERM P-18285 with International Patent Organism Depository (IPOD), National Institute of Advanced Industrial Science and Technology (AIST), Japan (Tsukuba City, Higashi 1-1-1, Ibaraki Prefecture, Japan) on April 2, 2001. Later, on Februaby 28, 2002, the same strain has been deposited as FERM BP-7931 based on Budapest convention.

0.52g of 98% sulfuric acid was added to 600ml of the enzyme reaction solution which contains 43.8g of 4-aminobutylguanidine obtained by the above-mentioned method, it was adjusted to be weak acidic, and then, sterilization by heating and coagulation were carried out at 120°C for 10 minutes. After cooling the heat treated solution to an ordinary temperature, the microorganism was removed by centrifugal operation. Water was added to the supernatant which was obtained here and the microorganism cake. After re-slurry, centrifugal operation was carried out, the supernatant which was obtained was added thereto, and 1000ml of a dezymotized solution containing 42.8g of 4-aminobutylguanidine was obtained. 0.43ml of 50% benzalconium chloride aqueous solution was added to the dezymotized solution to be adequately mixed, protein coagulation was carried out, then 2.14g of active carbon was added thereto, and the excessive benzalconium chloride was adsorbed. The coagulated substance and active carbon were filtered to obtain 970ml of purified solution containing 38.5g of 4-aminobutylguanidine. Further, the solution was concentrated to 640ml to prepare the 6% 4-aminobutylguanidine aqueous solution.

### (2) Production of lauroylamidobutylguanidine

180g of water and 110g of 2-propanol were added to 52.7g (0.23mol) of 4-aminobutylguanidine sulfate which was obtained by concentrating and drying the 4-aminobutylguanidine aqueous solution which was produced by the method (1), and the pH of the system was adjusted to be 11.0 and the temperature was adjusted to be 10°C using a 27% sodium hydroxide aqueous solution. In this system, 50.3g (0.23mol) of lauroyl chloride was added drop-by-drop for a period of about 30 minutes. At this time, the temperature of the system was maintained at 8 to 12°C, and pH was maintained at 10.9 to 11.0 by the 27% sodium hydroxide aqueous solution. After the completion of the addition of lauroyl chloride, the reaction was further ripened for a period of about 30 minutes.

After completion of the reaction, the reaction system was separated to an oil layer and a water layer by heating to 50°C, the 27% sodium hydroxide was added to the oil layer which was separated, to adjust pH to be 14.0, and then the system was cooled to the room temperature. The solid precipitated was separated by filtration, and dried after being further washed with water.
Light yellow solid, 65.8g (yield 92%)
¹H-NMR (CD30D) : σ = 0.90 (3H, t), 1.28-1.38 (18H, m), 1.57 (6H, m), 2.17 (2H, t), 3,19 (4H, m)
ESI-Mass: 313 (MH+)

A peak was confirmed by High Speed Liquid Chromatography (column: YMC-Pack ODS-AM AM-312, S-5µm, 120A, 150*6.0mm, column temperature: 40°C, eluant: 30mM phosphoric acid buffer (pH3.0)/methanol = 25/75, flow rate: 1.0ml/min., detection: UV210nm).

It was confirmed by Ion Chromatography that the amount of residual sulfuric acid was 0.12% (column: Ion pac AS-11 HC 2mm (Dionex), guard column: Ion pac AG-11 HC 2mm (Dionex), column temperature: room temperature, eluant: NaOH aqueous solution 1.5mM (0min) → 30mM (20min), flow rate: 0.38ml/min., regenerated liquid: 5mM H₂SO₄ about 3mL/min., detection: conductivity).

### Example 2:

### Production of myristoylamidobutylguanidine

180g of water and 165g of 2-propanol were added to 52.7g (0.23mol) of 4-aminobutylguanidine sulfate which was obtained by concentrating and drying the 4-aminobutylguanidine aqueous solution which was produced by the method of Example 1(1), and the pH of the system was further adjusted to be 11.0 and the temperature was adjusted to be 10°C by a 27% sodium hydroxide aqueous solution. In this system, 58.6g (0.15mol) of myristoyl chloride was added drop-by-drop for a period of about 30 minutes . At this time, the temperature of the system was maintained at 8 to 12°C, and pH was maintained at 10.9 to 11.0 using the 27% sodium hydroxide aqueous solution. After the completion of the addition of myristoyl chloride, the reaction was further ripened for a period of about 30 minutes.

After completion of the reaction, the reaction system was separated to an oil layer and a water layer by heating to 50°C, the 27% sodium hydroxide was added to the oil layer which was separated, to adjust pH to be 14.0, and then the system was cooled to a room temperature. The solid precipitated was separated by filtration, and dried after being further washed with water.
Light yellow solid, 72.7g (yield 96%)
¹H-NMR (CD30D) : σ = 0. 90 (3H, t), 1.28-1.38 (20H, m), 1.57 (6H, m), 2.17 (2H, t), 3,19 (4H, m)
ESI-Mass: 341 (MH+)

A peak was confirmed by High Speed Liquid Chromatography (column: YMC-Pack ODS-AM AM-312, S-5µm, 120A, 150*6.0mm, column temperature: 40°C, eluant: 30mM phosphoric acid buffer (pH3.0) /methanol = 25/75, flow rate: 1.0ml/min., detection: UV210nm).

It was confirmed by Ion Chromatography that the amount of residual sulfuric acid was 0.5% (column: Ion pac AS-11 HC 2mm (Dionex), guard column: Ion pac AG-11 HC 2mm (Dionex), column temperature: room temperature, eluant: NaOH aqueous solution 1.5mM (0min) → 30mM (20min), flow rate: 0.38ml/min., regenerated liquid: 5mM H₂SO₄ about 3mL/min., detection: conductivity).

### Example 3:

### Production of palmitoylamidobutylguanidine

361g of water and 165g of 2-propanol were added to 52.7g (0.23mol) of 4-aminobutylguanidine sulfate which was obtained by concentrating and drying the 4-aminobutylguanidine aqueous solution which was produced by the method of Example 1(1), and the pH of the system was adjusted to be 11.0 and the temperature was adjusted to be 10°C using a 27% sodium hydroxide aqueous solution. In this system, 63.4g (0.23mol) of palmitoyl chloride was added drop-by-drop for a period of about 30 minutes . At this time, the temperature of the system was maintained at 8 to 12°C, and pH was maintained at 10.9 to 11.0 using the 27% sodium hydroxide aqueous solution. After completion of the dropwise addition, the reaction was further ripened for a period of about 30 minutes.

After completion of the reaction, 80g of 2-propanol was additionally added to the reaction system, it was separated to an oil layer and a water layer by heating to 60°C, the 27% sodium hydroxide was added to the oil layer which was separated, to adjust pH to be 14.0, and then the system was cooled to a room temperature. The solid precipitated was separated by filtration, and dried after being further washed with water.
Light yellow solid, 77.9g (yield 92%)
¹H-NMR (CD30D): σ = 0.90 (3H, t), 1.28-1.38 (22 H, m), 1.57 (6H, m), 2.17 (2H, t), 3,19 (4H, m)
ESI-Mass: 369 (MH+)

A peak was confirmed by High Speed Liquid Chromatography(column:YMC-Pack ODS-AM AM-312, S-5µm, 120A, 150*6.0mm, column temperature: 40°C, eluant: 30mM phosphoric acid buffer (pH3.0)/methanol = 15/85, flow rate: 1.0ml/min., detection: UV210nm).

It was confirmed by Ion Chromatography that the amount of residual sulfuric acid was 0.04% (column: Ion pac AS-11 HC 2mm (Dionex), guard column: Ion pac AG-11 HC 2mm (Dionex), column temperature: room temperature, eluant: NaOH aqueous solution 1.5mM (0min) → 30mM (20min), flow rate: 0.38ml/min., regenerated liquid: 5mM H₂SO₄ about 3mL/min., detection: conductivity).

### Example 4:

### Synthesis of lauroylamidobutylguanidine acetate

The pH of the system was adjusted to be 11.0 and the temperature was adjusted to be 10°C by adding a 27% sodium hydroxide aqueous solution to 500ml of a 0.24mol aqueous solution of the 4-aminobutylguanidine sulfate which was obtained in like manner as in Example 1(1). In this system, 52g (0.24mol) of lauroyl chloride was added drop-by-drop for a period of about 20 minutes. At this time, the temperature of the system was maintained at 8 to 12°C, and pH was maintained at 10.9 to 11.0 using the 27% sodium hydroxide aqueous solution. After completion of the dropwise addition, the reaction was further ripened for a period of about 30 minutes.

After completion of the reaction, the solid precipitated was separated by filtration. 200ml of a hydrochloric acid methanol solution (content of hydrochloric acid is 10 to 20% by weight) was charged to the solid which was separated by filtration, to adjust pH to be 1.0, and the mixture was maintained at 50°C for a period of about 30 minutes. Then, 15g (0.26mol) of acetic acid was added thereto, 100g of a 17wt% KOH ethanol solution was charged, and the mixture was stirred to be pH of 7 and be cooled to a room temperature. After letting it stand alone, the solid precipitated was separated by filtration, and the solvent of the mother liquid was distilled off . The residue was recrystallized from butanone.
White solid, 37g (yield 44%)
¹H-NMR (CD30D) : σ = 0.90 (3H, t), 1.28-1.38 (18H, m), 1.57 (6H, m), 1.88 (3H,s), 2.17 (2H, t), 3,19 (4H, m)

A peak was confirmed by High Speed Liquid Chromatography (column: YMC-Pack ODS-AM AM-312, S-5µm, 120A, 150*6.0mm, column temperature: 40°C, eluant: 30mM phosphoric acid buffer (pH3.0)/methanol = 25/75, flow rate: 1.0ml/min., detection: UV210nm).

According to the production process of the present invention, the objective guanidine derivative containing an amido group and its salt can be industrially advantageously and simply produced.

Further, since the complicated synthetic steps required for the synthesis of a guanidine derivative containing an amido group can be simplified by using the production process of the present invention, the cost down of the guanidine derivative containing an amido group which is useful as a surfactant can be attained.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

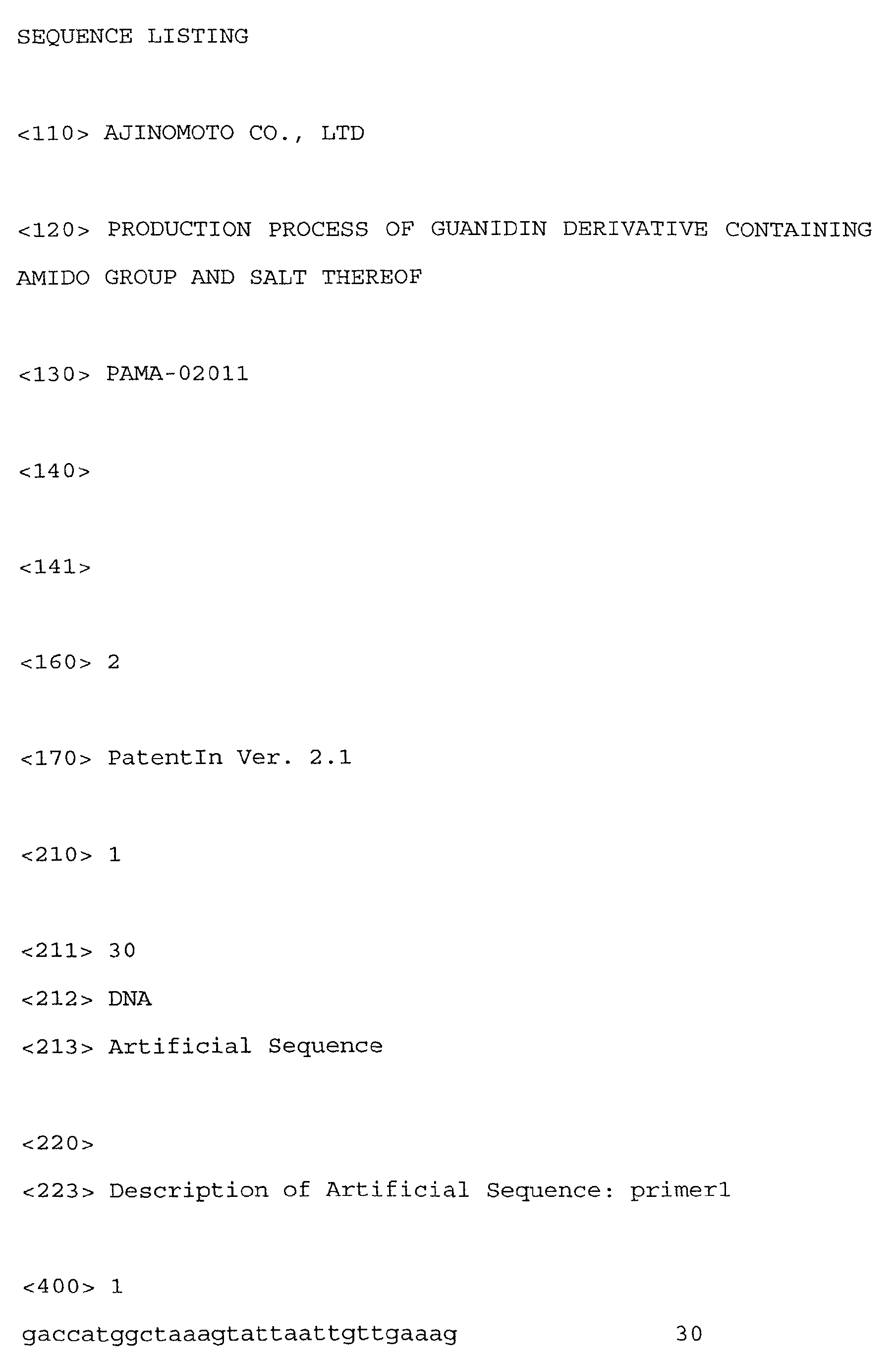

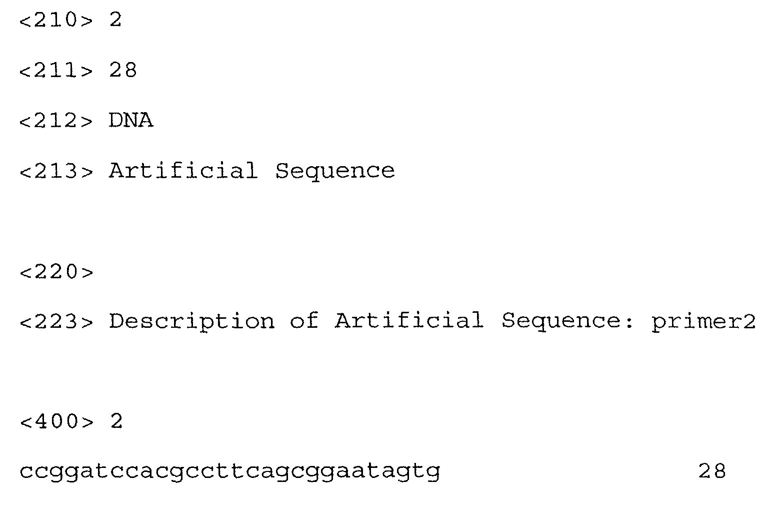

## Claims

1. A production process of a guanidine derivative containing an amido group represented by a general formula (I) 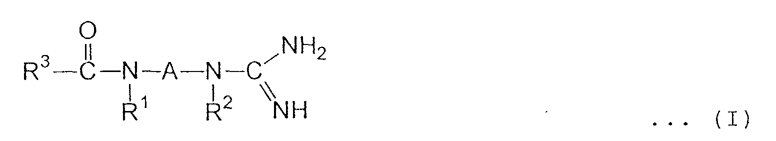 where R¹ and R² are each chosen from a group consisting of hydrogen atoms, a linear chain or branched chain alkyl group having 1 to 4 carbons, and a linear chain or branched chain alkenyl group having 1 to 4 carbons and R¹ and R² are have same or different composition, R³ is a linear chain or a branched chain alkyl group or an alkenyl group having 1 to 22 carbons, A is a linear chain or branched chain alkylene group or alkenylene group having 1 to 10 carbons, comprising a step of:
allowing a guanidine derivative containing an amino group represented by the general formula (II) to react with a fatty acid halide or a fatty acid ester, 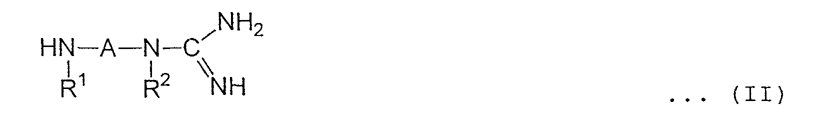
where R¹ and R² are each selected from the group consisting of hydrogen atoms, a linear chain or branched chain alkyl group having 1 to 4 carbons, and a linear chain or branched chain alkenyl group having 1 to 4 carbons and R¹ and R² are have same or different composition, A is a linear chain or a branched chain alkylene group or an alkenylene group having 1 to 10 carbons.

2. The production process according to claim 1, wherein R³ in the general formula (I) is a linear chain or branched chain alkyl group or alkenyl group having 7 to 17 carbons.

3. The production process according to claim 1, wherein the guanidine derivative represented by the general formula (II) is 4-aminobutylguanidine.

4. The production process according to claim 3, wherein the 4-aminobutylguanidine is obtained by decarboxylating arginine using Arginine decarboxylase.

5. The production process according to claim 4, wherein the Arginine decarboxylase is Arginine decarboxylase which was produced by a recombinant in which Arginine decarboxylase gene is amplified expressed.

6. The production process according to claim 5, wherein the Arginine decarboxylase gene is adi gene derived from Escherichia Coli.

7. The production process according to claim 5, wherein the recombinant is FERM-P18285 strain.

8. The production process according to claim 4, wherein the pH of reaction system is controlled at less than pH6 in a transformation reaction from arginine to 4-aminobutylarginine.

9. The production process according to claim 1, wherein the guanidine derivative containing an amino group represented by the general formula (II) is reacted with a fatty acid halide.

10. The production process according to claim 9, wherein the reaction of the guanidine derivative containing an amino group with the fatty acid halide is performed in water or a mix solvent of water and an organic solvent.

11. A production process of a salt of a guanidine derivative containing an amido group represented by a general formula (I) 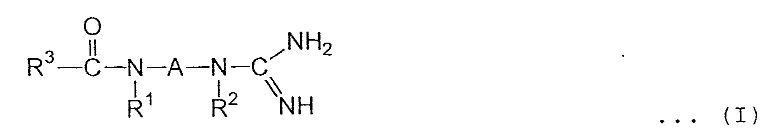 where R¹ and R² are each chosen from a group consisting of hydrogen atoms, a linear chain or branched chain alkyl group having 1 to 4 carbons, and a linear chain or branched chain alkenyl group having 1 to 4 carbons and R¹ and R² are have same or different composition, R³ is a linear chain or a branched chain alkyl group or an alkenyl group having 1 to 22 carbons, A is a linear chain or branched chain alkylene group or alkenylene group having 1 to 10 carbons, comprising steps of:
producing a solution of a guanidine derivative by allowing a guanidine derivative containing an amino group represented by the general formula (II) to react with a fatty acid halide or a fatty acid ester 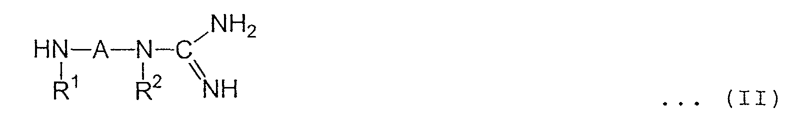
where R¹ and R² are each selected from the group consisting of hydrogen atoms, a linear chain or branched chain alkyl group having 1 to 4 carbons, and a linear chain or branched chain alkenyl group having 1 to 4 carbons and R¹ and R² are have same or different composition, A is a linear chain or a branched chain alkylene group or an alkenylene group having 1 to 10 carbons; and
neutralizing the solution of the guanidine derivative.
